Europäisches Patentamt

European Patent Office

Office européen des brevets

(11) Veröffentlichungsnummer: **0 183 956 B1**

(12) **EUROPÄISCHE PATENTSCHRIFT**

(45) Veröffentlichungstag der Patentschrift: **03.04.91**

(51) Int. Cl.⁵: **A61L 2/06, //A61J9/00**

(21) Anmeldenummer: **85113406.4**

(22) Anmeldetag: **22.10.85**

(54) **Gerät zum Sterilisieren von Babymilchflaschen.**

(30) Priorität: **02.11.84 DE 3440146**

(43) Veröffentlichungstag der Anmeldung:
**11.06.86 Patentblatt 86/24**

(45) Bekanntmachung des Hinweises auf die
Patenterteilung:
**03.04.91 Patentblatt 91/14**

(84) Benannte Vertragsstaaten:
**AT BE FR GB IT LU NL SE**

(56) Entgegenhaltungen:
**DE-A- 3 147 301
DE-A- 3 332 172
US-A- 2 467 337**

(73) Patentinhaber: **DAVID + BAADER - DBK - Spezialfabrik Elektrischer Apparate und Heizwiderstände - GmbH
Rheinstrasse 72-74
W-6744 Kandel/Pfalz(DE)**

(72) Erfinder: **David, Josef
Maxburgring 7 b
W-6748 Bad Bergzabern(DE)**

(74) Vertreter: **Patentanwälte Grünecker, Kinkeldey, Stockmair & Partner
Maximilianstrasse 58
W-8000 München 22(DE)**

Rank Xerox (UK) Business Services

## Beschreibung

Die vorliegende Erfindung bezieht sich auf ein Gerät zum Sterilisieren von einer oder mehreren Babymilchflaschen nach dem Oberbegriff des Patentanspruchs 1. Ein solches Gerät ist aus der DE-OS 31 49 754 bekannt.

Das bekannte Gerät basiert auf einem handelsüblichen Haushaltseierkochgerät und gestaltet dieses durch Zusatzteile, bestehend aus einem auf den Eierhalteeinsatz aufzusetzenden Flaschenhalter und einer der Höhe der Milchflaschen angepaßten, auf das Heizunterteil aufzusetzenden Haube zu einem Sterilisiergerät für Babymilchflaschen um. Nachteilig an diesem Gerät ist, daß wegen der relativ großen Bauhöhe der Haube und der nur bescheidenen Verdampfungsleistung des Eierkochgerätes im oberen Bereich der Haube eine Rekondensation des Wasserdampfes stattfindet, so daß mit einer gleichmäßigen Temperaturverteilung und Sterilisierung der Flaschen nicht zu rechnen ist. Darüberhinaus sind die Flaschen, wenn man die Haube abnimmt, völlig ungeschützt, sie können leicht von dem Flaschenhalter herunterfallen, und der Flaschenhalter selbst kann nur sehr schwierig ergriffen werden. Im heißen Zustand ist das Gerät, angefangen bei der Haube, nicht manipulierbar.

Aus der DE-OS 31 29 897 ist eine Vorrichung zum Auskochen von Säuglingsflaschen bekannt, die als ein in einen Kochkessel hineinstellbarer Einsatz ausgebildet ist, der auf einer durchbrochenen Bodenplatte Aufnahmevorrichtungen für von oben einsetzbare Babymilchflaschen hat. Dieser Einsatz wird im Gebrauch nach dem Sterilisieren der Babymilchflaschen mit diesen beladen aus dem Kochkessel herausgenommen, so daß in diesem Zustand die Flaschen wiederum ungeschützt und Keimen zugänglich sind.

Aus dem DE-GBM 80 21 633 ist eine Vorrichtung zum Sterilisieren von Babymilchflaschen bekannt, bestehend aus einer Wasserschale, unter deren Boden Heizelemente angebracht sind, einem darüber angeordneten Halter für Babymilchflaschen, der sich etwa über die gesamte Höhe der Milchflaschen erstreckt und diese sicher hält, und einer die Gesamtanordnung abdeckenden Haube. Die Haube ist nach außen nicht isoliert, so daß an ihr, besonders im oberen Bereich, eine Rekondensation von Wasserdampf auftreten kann. Die Haube wird daher im Betrieb relativ heiß und kann mit ungeschützten Händen nicht angegriffen werden. Nachteilig ist ferner, daß beim Herausnehmen des Einsatzes aus dem Gerät die Flaschen ungeschützt der Umgebungsluft ausgesetzt sind.

Der Erfindung liegt die Aufgabe zugrunde, ein Gerät der eingangs genannten Art anzugeben, das eine möglichst gleichmäßige Wärmeverteilung in dem dampferfüllten Raum gewährleistet, und das auch im Betrieb oder unmittelbar danach auch mit ungeschützten Händen manipulierbar ist.

Diese Aufgabe wird durch die kennzeichnenden Merkmale des Patentanspruchs 1 gelöst. Eine Porteilhafte Ausgestaltung der Erfindung ist im Anspruch 2 angegeben.

Die Erfindung schafft ein Gerät zum Sterilisieren von Babymilchflaschen, bei dem das Heizunterteil fast nicht mehr als Unterteil bezeichnet werden kann, da es sich bis über die Hälfte der Gesamthöhe des Gerätes erstrecken kann. In dieses Heizunterteil ist der topfförmig gestaltete Einsatz einsetzbar, wobei dieser zu dem Mantel des Heizunterteils einen Abstand hält, der eine hervorragende Isolation des dampferfüllten Raumes gegenüber der Umgebung sicherstellt. Es wird hierdurch nicht nur eine Vergleichmäßigung der Temperatur in dem dampferfüllten Raum erreicht, sondern es wird auch vermieden, daß die Außenwand des Gerätes sich so weit aufheizt, daß es nicht mehr mit der Hand angefaßt werden kann.

Ein besonderer, sehr wesentlicher Vorteil des erfindungsgemäßen Gerätes besteht weiterhin darin, daß der Einsatz mit darauf befindlicher Haube aus dem Heizunterteil herausgenommen und zum Abkühlen abgestellt werden kann, ohne daß dadurch die Flaschen sofort der Umgebungsatmosphäre ungeschützt ausgesetzt werden.

Zum Erleichtern des Herausnehmens des Einsatzes ist dieser mit einer Griffeinrichtung versehen, die von einem hochgezogenen Kragen gebildet ist. Die Haube ist im Abstand zu diesem Kragen in diesen eingesetzt, wobei der Abstand zwischen der Haube und dem Kragen es verhindert, daß sich die Wärme der Haube auf den Kragen überträgt, so daß dieser ausreichend kühl bleibt, um noch mit ungeschützter Hand angefaßt werden zu können.

Die Erfindung soll nachfolgend unter Bezugnahme auf ein in den Zeichnungen dargestelltes Ausführungsbeispiel näher erläutert werden. Es zeigt:

Fig. 1      eine Seitenansicht eines erfindungsgemäßen Gerätes im Schnitt,

Fig. 2      einen Querschnitt durch das Gerät nach Fig. 1 längs der Linie II-II,

Fig. 3      im Ausschnitt eine Seitenansicht im oberen Bereich eines Gerätes mit einem Haltering im Schnitt, und

Fig. 4      den Haltering in Fig. 3 von oben.

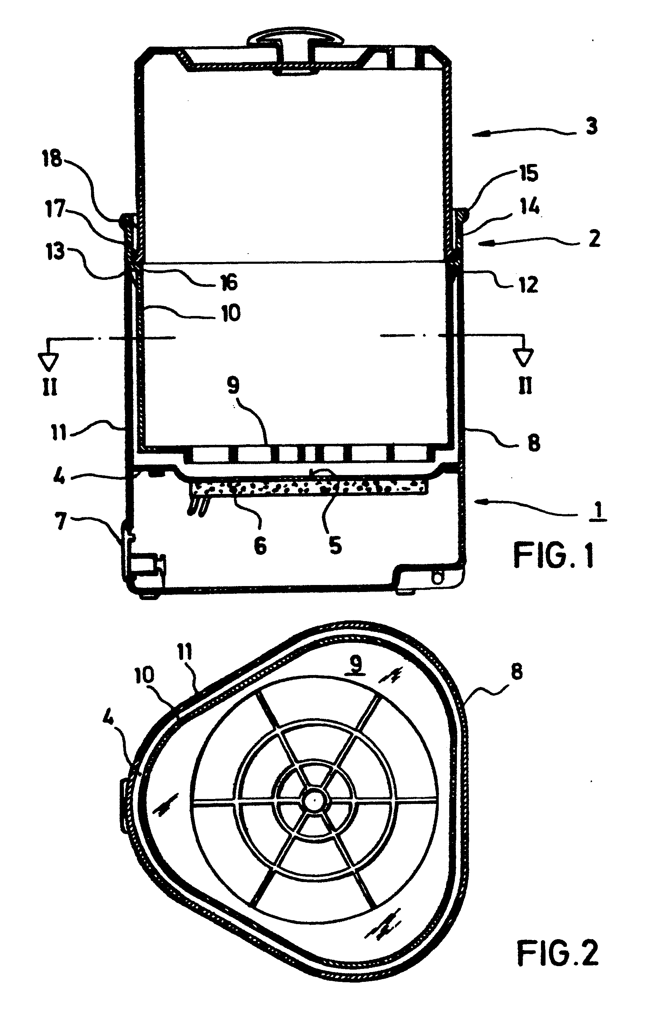

Das Gerät nach Fig. 1 hat, wie Fig. 2 zeigt, einen etwa dreieckförmigen Grundriß mit abgerundeten Ecken, da diese Querschnittsform zur Unterbringung von Babymilchflaschen besonders zweckmäßig ist. Das Gerät besteht im wesentlichen aus einem Heizunterteil 1, einen darin einsetzbaren Einsatz 2 und einer Haube 3. Das Heizunterteil 1 enthält einen Innenboden 4 mit einer davon gehaltenen Kochschale 5, die von der Unterseite mittels einer elektrischen Heizeinrichtung 6 beheizbar ist. Ein Schalter 7 im unteren Bereich der Wand des Heizunterteils 1 dient dem Einschalten der Stromversorgung der elektrischen Heizeinrichtung 6. Die elektrische Verdrahtung ist aus Übersichtlichkeitsgründen nicht dargestellt und ist zur Erläuterung der Erfindung nicht erforderlich. Das Heizunterteil 1 weist eine Seitenwand oder einen Mantel 8 auf, der sich bis über die Hälfte der Gesamthöhe des Gerätes erstreckt. Die Höhe dieses Mantels ist so gewählt, daß sie, etwa vom Niveau der Kochschale 5 ausgehend noch immer wenigstens etwa halb so hoch ist, wie die Höhe der von dem Gerät aufzunehmenden Flaschen.

Der Einsatz 2 zur Aufnahme der Babymilchflaschen ist etwa topfförmig gestaltet und weist einen durchbrochenen Boden 9 auf. Die Höhe des Einsatzes 2 ist so bemessen, daß dessen in das Heizunterteil 1 ragende Wand 10 dicht über dem Zwischenboden 4 endet. Wie die Figuren 1 und 2 zeigen, ist der Querschnitt des Einsatzes 2 in bezug auf den des Heizunterteils 1 so bemessen, daß nach dem Einsetzen des Einsatzes 2 in das Heizunterteil 1 zwischen der Innenseite des Mantels 8 und der Außenseite der Wand 10 ein Spalt 11 von wenigstens etwa 1 mm Dicke frei bleibt. Um allseitig einen definierten Wandabstand einzuhalten, sind an der Außenseite der Wand 10 mehrere ringsum verteilte Abstandshaltenasen 12 ausgebildet.

Der Einsatz 2 ist im Bereich seines oberen Endes mit einem nach außen ragenden Sitz 13 versehen, der sich auf dem Rand des Mantels 8 des Heizunterteils 1 abstützt. Von diesem Sitz ausgehend erstreckt sich nach oben ein Kragen 14, der an seinem oberen Rand von einer Randwulst 15 umgeben ist, die das Ergreifen erleichtert.

Im Bereich des Sitzes 13 weist der Einsatz 2 an seiner Innenwand einen Sitz 16 auf, der der Abstützung der Haube 3 dient.

Wesentliches Merkmal der Haube ist, daß sie an ihrem unteren Rand eine nach außen vorstehende Wulst 17 aufweist, mit deren Hilfe zwischen der Innenwand des Kragens 14 und der Außenwand der Haube, deren Querschnitt entsprechend kleiner dimensioniert ist, ein Spalt 18 verbleibt.

Die Breiten der Spalte 11 und 18 sind so bemessen, daß sich ein für Isolierzwecke ausreichend dickes Luftpolster zwischen den parallel laufenden Wänden bzw. dem Kragen und der Wand ergibt.

Im Betrieb wird auf die Kochschale 5 eine definierte Wassermenge gegeben, der mit Babymilchflaschen beladene Einsatz wird in das Heizunterteil eingesetzt und die Haube wird aufgesetzt. Sodann wird die Stromversorgung für die Heizeinrichtung 6 angeschaltet. Die Heizeinrichtung 6 bringt das Wasser in der Kochschale 5 zum Sieden, wodurch sich Dampf entwickelt. Der Dampf steigt durch den durchbrochenen Boden 9 in die Babymilchflaschen, die zweckmäßigerweise mit der Öffnung nach unten in den Einsatz 2 hineingestellt worden sind, und in den von dem Einsatz 2 und der Haube 3 umschlossenen Raum. Der Siedevorgang wird solange aufrechterhalten, bis sämtliches Wasser aus der Kochschale verdampft ist. Mittels eines Überhitzungsschalters (nicht dargestellt) kann dann die Stromversorgung für die Heizeinrichtung 6 auf bekannte Weise unterbrochen werden.

Es kann dann sofort anschließend, oder nach einer gewünschten Wartezeit, der Einsatz mit aufgesetzter Haube aus dem Heizunterteil herausgenommen werden, indem man ihn an dem Kragen 14 mit beiden Händen ergreift.

Infolge der Spalte 11 und 18 bleiben sowohl der Mantel 8 als auch der Kragen 14 vor Überhitzung geschlltzt, so daß dem Benutzer davor bewahrt wird, sich an dem Gerät zu verbrennen, wenn er im Betrieb mit ihm in Berührung kommt.

Es sei betont, daß die Abstand haltenden Elemente 12 und 17 abweichend von der dargestellten Art auch an dem jeweils anderen beteiligten Element ausgebildet sein können. Auch ist die Erfindung ebenso an Geräten mit kreisrundem Grundriß oder anders gestaltetem Grundriß anwendbar.

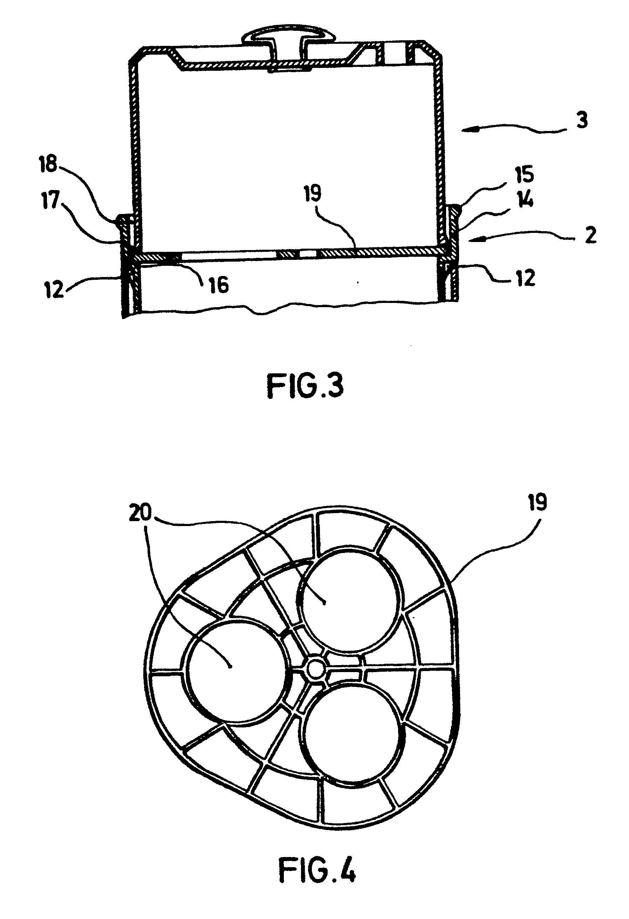

Es ist günstig, wenn man in dem Gerät zur Sicherung der Flaschen in ihrer aufrechten Stellung einen Haltering verwendet. Der Haltering sollte vom Boden des topfartigen Einsatzes einen ausreichenden Abstand aufweisen. Die Lage eines solchen Halterings ist in Fig. 3 dargestellt. Der Haltering 19, vorzugsweise aus einem gespritzten Kunststoffmaterial bestehend, weist, wie Fig. 4 zeigt, einen dem freien Innenquerschnitt des topfartigen Einsatzes 10 angepaßten Umriß auf, so daß er auf den Sitz 16 des topfartigen Einsatzes 10 aufgelegt werden kann, wie Fig. 3 zeigt. Über diesen Haltering 19 ist, wie zuvor, die Haube 3 aufsetzbar. Der Haltering 19 weist eine gitterartig durchbrochene Struktur auf mit dem Durchmesser der aufzunehmenden und zu haltenden Flaschen angepaßten Öffnungen 20. Der Haltering 19 ist ein separates Teil, das zur Reinigung aus dem Gerät herausgenommen werden kann.

Wenigstens der Einsatz besteht zweckmäßigerweise aus einem Wärme schlechtleitendem Material, etwa einem Kunststoffmaterial, das zugleich den Vorteil eines geringen Wärmeaufnahmevermögens hat. Auch die Haube und der Mantel können aus solchem Material bestehen.

Die Erfindung ermöglicht ein, je nach Wunsch langsames oder schnelles Abkühlen der Flaschen, wobei diese auch bei schnel lem Abkühlen, wenn der Einsatz aus dem Heizunterteil herausgenommen ist, vor direktem Kontakt mit der Umgebungsluft bewahrt sind. Ein Vorteil ist auch, daß die Flaschen in dem topfartigen Einsatz sicher gehalten sind, sie können nicht herunterfallen und sind daher vor Bruch geschützt. Die vorliegende Erfindung vereinigt somit in sich eine ganze Reihe von Vorteilen, die bei den Geräten nach dem Stand der Technik nicht zu finden sind.

## Ansprüche

1. Gerät zum Sterilisieren von einer oder mehreren Babymilchflaschen, bestehend aus einem Heizunterteil mit einer Kochschale zur Aufnahme und zum Verdampfen von Wasser, einem darüber angeordneten, sich auf dem Rand des Heizunterteils abstützenden, abnehmbaren Einsatz mit durchbrochenem Boden zur Aufnahme der Milchflasche (n) und gegebenenfalls von Saugern und einer darüber stülpbaren Haube, **dadurch gekennzeichnet, daß** das Heizunterteil (1) einen senkrecht auf der Kochschale (5) stehenden Mantel (8) aufweist, daß der Einsatz (2) topfformig gestaltet ist, wobei sein Querschnitt so dimensioniert ist, daß zwischen der Außenseite seiner Wand (10) und der Innenseite des Mantels (8) ein Spalt (11) von wenigstens 1 mm gebildet ist, daß die Wand des Einsatzes (2) oberhalb seines sich auf dem Mantel (8) des Heizunterteils (1) abstützenden Sitzes (13) mit einer von außen zugänglichen Griffeinrichtung (14) versehen ist, daß die Griffeinrichtung ein sich von dem Sitz (13) nach oben erstreckender Kragen (14) ist, daß der Kragen (14) auf seiner Innenseite im Bereich seines unteren Endes mit einem Sitz (16) für die Haube (3) versehen ist, daß der Umriß der Haube (3) ausreichend kleiner als der innere Umriß des Kragens (14) ist, daß zwischen beiden ein Spalt (18) von wenigstens 1 mm Breite gebildet wird, und daß an der Haube (3) und/oder an dem Kragen (14) Einrichtungen (17) zur Abstandshaltung von Haube (3) und Kragen (14) ausgebildet sind.

2. Gerät nach Anspruch 1, **dadurch gekennzeichnet, daß** an der Außenseite der Nand (10) des Einsatzes (2) oder an der Innenwand des Mantels (8) Abstandshalter (12) zur Abstandshaltung von Einsatz (2) und Mantel (8) ausgebildet sind.

## Claims

1. Apparatus for sterilising one or more baby's feeding bottles, consisting of a lower heating part with a boiling dish for receiving and evaporating water, of a removable insert bearing on the edge of the lower heating part with an apertured base for receiving the feeding bottle(s) and optionally teats, and of a hood which can be placed on top, characterised in that the lower heating part (1) has a casing (8) standing vertically on the boiling dish (5), in that the insert (2) is pot-shaped, its cross-section being so dimensioned that between the outside of its wall (10) and the inside of the casing (8) a gap (11) of at least 1 mm is formed, in that the wall of the insert (2) is provided above its seat (13), which bears on the casing (8) of the lower heating part (1), with a handle device (14) which is accessible from outside, in that the handle device is a collar (14) extending upwards from the seat (13), in that the collar (14) is provided on its inside in the region of its lower end with a seat (16) for the hood (3), in that the outline of the hood (13) is sufficiently smaller than the inner outline of the collar (14), so that between the two a gap (18) of at least 1 mm wide is formed, and in that on the hood (3) and/or on the collar (14) devices (17) for spacing the hood (3) and the collar (14) are formed.

2. Apparatus according to claim 1, characterised in that on the outside of the wall (10) of the insert (2) or on the inner wall of the casing (8) spacers (12) are formed to retain the distance between the insert (2) and the casing (8).

## Revendications

1. Appareil pour stériliser un ou plusieurs biberons, comprenant une partie inférieure chauffante avec un bac d'ébullition pour recevoir et vaporiser de l'eau, un insert amovible avec fond percé qui est disposé dessus, prend appui su le bord de la partie inférieure chauffante et est destineé à recevoir un(des) biberon(s) et éventuellement des tétines et un capuchon pouvant être placé sur ledit insert, caractérisé, en ce que la partie inférieure chauffante (1) présente une enveloppe (8) Placée verticalement sur le bac d'ébullition (5), en ce que l'insert (2) est réalisé en force de pot, sa section transversale étant dimensionnée de telle manière qu'une fente (11) d'au moins 1 mm soit formée entre le côté extérieur de sa paroi (10) et le côté intérieur de l'enveloppe (8), en ce que au-dessus de son appui (13) reposant sur l'enveloppe (8) de la partie inférieure chauffante (1), la paroi de l'insert (2) est munie d'un dispositif de préhension (14) accessible de l'extérieur, en ce que le dispositif de préhension est une collerette (14) s'étendant vers le haut à partir de L'appui (13), en ce que la collerette (14) est munie sur son côté intérieur, dans la zone de son extrémité inférieure, d'un appui (16) pour le capuchon (3), en ce que le contour du capuchon (3) est suffisamment inférieur au profil intérieur de la collerette (14) pour qu'une fente (18) d'une largeur d'au moins 1 mm soit formée entre les deux et en ce que des dispositifs (17) pour écarter le capuchon (3) et la collerette (14) sont réalisés sur le capuchon (3) et/on sur la collerette (14).

2. Appareil selon la revendication 1, caractérise en ce que des dispositifs d'espacement (12) pour écarter l'insert (2) de l'enveloppe (8) sont réalisés sur le côté extérieur de la paroi (10) de l'insert (2) ou sur la paroi intérieure de l'enveloppe (8).

FIG.1

FIG.2

FIG.3

FIG.4